# FASCICULE DE BREVET EUROPEEN

(11) **EP 1 094 049 B1**
(45) Date de publication et mention de la délivrance du brevet: **16.04.2003**
(21) Numéro de dépôt: 00402496.4
(22) Date de dépôt: 11.09.2000
(51) Int. Cl.: C07C 1/04, C07C 9/14, C07C 1/06, C10G 2/00, B01J 25/00

(54) **Procédé de synthèse d'hydrocarbures à partir de gaz de synthèse en présence d'un alliage métallique de Raney dispersé dans une phase liquide**
Verfahren zur Synthese von Kohlenwasserstoffen aus Synthesegas in Gegenwart von einer, in einer flüssigen Phase dispergierten Raney Metalllegierung
Process for synthesising hydrocarbons from synthesis gas in the presence of a Raney metal alloy dispersed in a liquid phase

(30) Priorité: 22.10.1999 FR 9913325
(43) Date de publication de la demande: 25.04.2001
(73) Titulaire: INSTITUT FRANCAIS DU PETROLE, 92852 Rueil-Malmaison Cedex (FR); ENI S.p.A., 00144 Roma (IT)
(72) Inventeur: Hugues, François, Charly, 69390 Vernaison (FR); Roy-Auberger, Magalie, 92500 Rueil-Malmaison (FR); Marion, Marie-Claire, 69390 Vernaison (FR)

(56) Documents cités:
- EP-A- 0 087 771

## Description

La présente invention concerne un procédé de synthèse d'hydrocarbures à partir d'un mélange comprenant CO-(CO₂)-H₂ (c'est à dire un mélange comprenant du monoxyde de carbone et de l'hydrogène et éventuellement du CO₂, communément appelé gaz de synthèse). Ce procédé de conversion du gaz de synthèse comprend la mise en oeuvre d'un catalyseur particulier, habituellement appelé catalyseur de Raney.

Ce type de catalyseur est essentiellement constitué par un alliage métallique comprenant au moins 60% poids d'au moins un métal choisi parmi les métaux des groupes 8, 9 ou 10. Il peut toutefois comprendre d'autres constituants détaillés ci-après.

Dans le procédé selon l'invention, ce catalyseur est mis en oeuvre dans un réacteur opérant en phase liquide, de préférence un réacteur slurry , c'est-à-dire un réacteur triphasique opérant avec le catalyseur solide en suspension dans une phase liquide et en présence d'une phase gazeuse. De manière plus préférée on met en oeuvre le catalyseur dans un réacteur slurry de type colonne à bulle triphasique (slurry bubble column).

### ART ANTERIEUR :

II est connu de l'homme du métier que le gaz de synthèse peut être converti en hydrocarbures en présence de catalyseurs contenant des métaux de transition. Cette conversion opérée à haute température et sous pression est connue dans la littérature sous le nom de synthèse Fischer-Tropsch. Ainsi des métaux des groupes 8, 9 ou 10 de la classification périodique des éléments tels que par exemple le fer, le ruthénium, le cobalt et le nickel catalysent la transformation de mélanges CO-(CO₂)-H₂ (c'est-à-dire un mélange CO-H₂ comprenant éventuellement du CO₂, appelé gaz de synthèse) en hydrocarbures liquides et/ou gazeux.

Différentes méthodes ont été décrites dans l'art antérieur, qui sont destinées à améliorer la préparation de catalyseurs Fischer-Tropsch à base de fer ou de cobalt supporté sur différents supports. Les supports les plus utilisés sont l'alumine, la silice et le dioxyde de titane.

Les catalyseurs de type cobalt ou nickel de Raney sont depuis longtemps connus de l'homme du métier en tant que catalyseurs utilisables dans des réactions d'hydrogénation de composés organiques.

Le procédé Raney décrit la façon de préparer un catalyseur métallique poreux et actif en préparant d'abord un alliage bimétallique où l'un des deux métaux peut être extrait pour donner un matériau poreux non soluble et actif en catalyse. (brevets US-A-1 628 190, US-A-1 915 473 et US-A-2 977 327).

Un catalyseur dit de Raney est un catalyseur formé de métal insoluble, bien connu dans le processus de Raney et qui typiquement peut être le nickel, le cobalt, le cuivre ou le fer.

Les catalyseurs de Raney sont en général produits à partir d'un alliage du métal catalytique considéré (par exemple le nickel ou le cobalt) avec l'aluminium. L'alliage est réduit en poudre, puis l'aluminium est éliminé par attaque au moyen d'une solution de soude, faisant ainsi apparaître un métal finement divisé, qui présente une surface spécifique généralement comprise entre 10 et 150 m2/g, et plus préférentiellement entre 10 et 100 m2/g. Sous cette forme le métal obtenu (nickel ou cobalt) présente une grande capacité d'adsorption d'hydrogène, d'où son intérêt en catalyse.

Le brevet EP 0 648 534 décrit la préparation de métaux de Raney mis en forme pour leur utilisation en lit fixe. Ces métaux peuvent être utilisés en tant que catalyseur d'hydrogénation de composés organiques.

Le brevet US 4 895 994 décrit la mise en forme de catalyseur contenant 15-50% en poids de métaux de Raney, un polymère et éventuellement d'autres additifs. Ces catalyseurs sont notamment utilisables pour la réaction d'hydrogénation du monoxyde de carbone.

Le brevet EP 0 450 861 décrit l'utilisation d'un procédé en phase slurry avec un catalyseur à base de cobalt supporté sur TiO₂. La mise en oeuvre d'une colonne à bulle dans lequel le catalyseur est mis en suspension dans une phase liquide permet d'obtenir au moins la productivité d'un réacteur en lit fixe, et la sélectivité d'un réacteur parfaitement agité.

### DESCRIPTION DE L'INVENTION :

La présente invention concerne un procédé de synthèse d'hydrocarbures à partir d'un mélange comprenant du monoxyde de carbone et de l'hydrogène CO-H₂, éventuellement du dioxyde de carbone CO₂, en présence d'un catalyseur essentiellement constitué par un alliage métallique comprenant au moins un métal choisi parmi les métaux des groupes 8, 9 ou 10, de préférence le fer ou le cobalt, utilisé en suspension dans une phase liquide dans un réacteur opéré le plus souvent en présence de trois phases : une phase liquide, une phase gazeuse et une phase solide au moins en partie constituée par le catalyseur (réacteur slurry).

Le catalyseur utilisé dans le procédé selon l'invention, est essentiellement constitué d'un alliage métallique contenant au moins environ 60 % poids d'au moins un métal choisi parmi les métaux des groupes 8, 9 ou 10, de préférence au moins 70% poids d'au moins un métal choisi parmi les métaux des groupes 8, 9 ou 10, et de manière très préférée au moins 80 % poids d'au moins un métal choisi parmi les métaux des groupes 8, 9 ou 10.

Le catalyseur présente une taille de grain généralement inférieure à environ 700 microns, de préférence inférieure à environ 250 microns, de manière plus préférée comprise entre 1 et 150 microns, et de manière très préférée comprise entre 10 et 80 microns, pour une utilisation optimale du réacteur opérant en phase liquide, notamment lorsqu'il s'agit d'un réacteur slurry de type colonne à bulle triphasique (slurry bubble column).

Le catalyseur utilisé dans le procédé selon l'invention peut être préparé au moyen de toute technique connu de l'homme du métier pour préparer des alliages de Raney. En particulier, il peut être préparé au moyen des méthodes décrites dans les brevets US-A-1 628 190, US-A-1 915 473, US-A-2 977 327 et EP-A- 0 648 534. Ce méthodes peuvent éventuellement être modifiées au moyen des connaissances de l'homme du métier, afin d'obtenir les caractéristiques souhaitées.

Une des méthodes préférées de préparation de ce catalyseur consiste en la formation d'un alliage entre les deux métaux, par exemple le cobalt et aluminium, à haute température (1300°C), la mise en forme par atomisation, puis l'activation par une solution d'hydroxyde de sodium pour donner après séparation de la solution d'aluminate de sodium, le catalyseur prêt à l'utilisation.

Le catalyseur peut de plus éventuellement contenir au moins un élément additionnel choisi parmi les métaux alcalins ou les métaux des groupes 4, 5, 6, 7, 8, 9, 10 ou 11 de la nouvelle classification périodique. Cet élément additionnel est de préférence choisi dans le groupe constitué par le titane, le zirconium, le fer, le ruthénium, le molybdène, le tungstène et le tantale.

La teneur en poids d'un élément additionnel par rapport au poids total de catalyseur est de préférence comprise entre 0 et environ 12 % poids, de manière plus préférée entre 0,01 et 10 % poids, de manière très préférée entre 0,1 et 5 % poids. Ces éléments additionnels peuvent être introduits en même temps que le métal ou les métaux choisis parmi les éléments des groupes 8, 9 ou 10, ou lors d'au moins une étape ultérieure.

La présente invention concerne un procédé de conversion du gaz de synthèse, utilisant un catalyseur dont les performances sont particulièrement stables, et qui conduit à un mélange d'hydrocarbures essentiellement linéaires et saturés contenant généralement au moins 50 % poids d'hydrocarbures C5⁺ et généralement moins de 20 % poids de méthane, de préférence moins de 15 % poids de méthane par rapport à l'ensemble des hydrocarbures formés.

Ce procédé est opéré sous une pression totale habituellement comprise entre 0,1 et 15MPa, de préférence comprise entre 1 et 10 MPa, une température généralement comprise entre 150°C et 350°C, de préférence comprise entre 170°C et 300°C. La vitesse volumétrique horaire est habituellement comprise entre 100 et 2000 volumes de gaz de synthèse par volume de catalyseur et par heure et de préférence comprise entre 400 et 5000 volumes de gaz de synthèse par volume de catalyseur et par heure. Le rapport molaire H2/CO du gaz de synthèse est habituellement compris entre 1:2 et 5:1, de préférence compris entre 1,2 :1 et 2,5 :1

Les conditions de mise en oeuvre desdits catalyseurs dans le procédé selon l'invention sont généralement les suivantes :

Le catalyseur est généralement utilisé directement dans la réaction, sans traitement préalable, et sous forme d'une poudre fine calibrée (granulométrie inférieure à environ 700 microns environ) en présence d'une phase liquide. La phase liquide peut être constituée par au moins un hydrocarbure ayant au moins 5 atomes de carbones par molécules, de préférence au moins 10 atomes de carbones par molécules. De préférence, cette phase liquide est constituée essentiellement de paraffines, et de manière plus préférée de paraffines issues d'une synthèse Fischer-Tropsch. En général on choisira une coupe dont les points d'ébullition initiaux et finaux sont tels qu'elle se situe dans la gamme des kérosènes ou des gasoils.

Il peut également être modifié au moyen d'un pré traitement avant l'introduction dans le réacteur. Ce pré traitement peut être par exemple, soit:
- un lavage permettant d'enlever les traces de la solution d'activation
- une réduction effectuée en phase gazeuse ou en phase liquide à une température comprise entre 100°C et 600°C de préférence entre 150°C et 400°C, à une pression comprise entre 0,1 et 10 MPa et à une vitesse volumétrique horaire entre 100 et 40000 volumes de mélange par volume de catalyseur et par heure, de préférence entre 500 et 10000 volumes de mélange par volume de catalyseur et par heure .

Lorsque cette réduction est effectuée en phase liquide, le catalyseur est mis en suspension dans un solvant inerte, par exemple une coupe paraffinique comprenant au moins un hydrocarbure ayant au moins 5 atomes de carbone par molécules, de préférence au moins 10 atomes de carbones par molécules.

Le catalyseur est avantageusement employé dans un réacteur slurry de type colonne à bulles triphasique (slurry bubble column). Ce type de mise en oeuvre présente l'avantage, par rapport à d'autres mise en oeuvre comme par exemple le lit fixe, de conduire à :
- une utilisation optimale des performances du catalyseur (activité et sélectivité), par limitation des phénomènes diffusionnels intra-granulaires.
- une limitation très importante des effets thermiques dans le grain de catalyseur, qui est entouré d'une phase liquide.

Ce type de mise en oeuvre nécessite une séparation du catalyseur et des produits de réaction. Elle peut être réalisée dans le réacteur ou à l'extérieur du réacteur.

Dans le premier cas, des éléments filtrants sont interposés sur la ligne d'évacuation des produits du réacteur, et maintiennent tout le catalyseur dans le réacteur, ainsi qu'il est décrit dans la demande de brevet EP-A-0 609 079.

Dans le second cas, la suspension (slurry) est généralement mise en circulation, au moyen par exemple d'une pompe. Divers moyens de séparation peuvent être employés, ainsi qu'il est indiqué dans la demande de brevet WO 97/31.693.

Ces deux modes de séparation peuvent être employés pour la mise en oeuvre du catalyseur utilisé dans le procédé selon l'invention, mais il a été observé de manière générale que ce catalyseur peut être aisément séparé des produits de la réaction au moyen de techniques connues de l'homme du métier, telles que la filtration, la séparation centrifuge, la décantation, la séparation magnétique ou la séparation par hydrocyclone.

Par ailleurs, un des avantages du catalyseur utilisé dans le procédé selon l'invention est que sa forte teneur en métal conduit à une densité élevée et à une séparation efficace de la plus grande partie du catalyseur au moyen par exemple d'une simple décantation. Le catalyseur employé présente en effet le plus généralement une densité apparente supérieure à 1,2 kilogrammes par (kg/l), de préférence supérieure à 1,5 kg/l, et de manière très préférée supérieure à 1,8 kg/l.

En résumé l'invention concerne donc un procédé de synthèse d'hydrocarbures à partir de gaz de synthèse, en présence d'un catalyseur essentiellement constitué par un alliage métallique de Raney dispersé dans une phase liquide, dans lequel le catalyseur comprend au moins au moins 60 % poids d'au moins un métal choisi parmi les métaux des groupes 8, 9 ou 10. De préférence, ledit métal est choisi parmi le fer ou le cobalt.

Le catalyseur du procédé selon l'invention peut en outre comprendre au moins un élément additionnel choisi parmi les métaux alcalins et les métaux des groupes 4, 5, 6, 7, 8, 9, 10 ou 11. De préférence cet élément additionnel est choisi dans le groupe constitué par le titane, le zirconium, le fer, le ruthénium, le molybdène, le tungstène et le tantale. La teneur en poids de l'élément additionnel par rapport au poids total de catalyseur est comprise entre 0 et environ 12 % poids, de préférence entre 0,01 et 10 % poids. Et de manière plus préférée entre 1 % et 5 % poids. Généralement le catalyseur présente une taille de grain inférieure à environ 700 microns.

De préférence, la phase liquide utilisée dans le procédé selon l'invention est une coupe kérosène ou gasoil issue de la synthèse Fischer-Tropsch.

Le catalyseur peut éventuellement être modifié au moyen d'un pré traitement avant l'introduction dans le réacteur, par exemple une réduction effectuée à une température comprise entre 100°C et 600°C, à une pression comprise entre 0,1 et 10 MPa et à une vitesse volumétrique horaire entre 100 et 40 000 volumes de mélange par volume de catalyseur et par heure.

De préférence, le catalyseur est mis en oeuvre dans un réacteur opérant en phase liquide de type colonne à bulle triphasique (slurry bubble column).

Les exemples qui suivent illustrent l'invention.

### Exemple 1 (selon l'invention): catalyseur A

Un catalyseur A, du type cobalt de Raney commercialisé par la société Degussa sous la référence B2112Z, se présente sous forme de poudre pré activée et conservée dans l'eau, à l'abri de l'air. La poudre métallique présente une granulométrie comprise entre 1 et 130 microns.
La teneur en cobalt métallique du catalyseur A est supérieure à environ 95% poids.

### Exemple 2 (selon l'invention): catalyseur B

Un catalyseur B, du type cobalt de Raney commercialisé par la société Degussa sous la référence Cobalt beta-1000, se présente sous forme de pastilles de taille 4 millimètres par 4 millimètres.
Un broyage humide de ces pastilles qui sont conservées dans de l'eau est effectué de façon à diminuer la granulométrie du catalyseur tout en le conservant à l'abri de l'air. La poudre récupérée présente une granulométrie comprise entre 1 et 130 microns. La teneur en cobalt métallique du catalyseur B est d'environ 60 % poids.

### Exemple 3 (comparatif) : catalyseur C

Un catalyseur C de formule Co/Al2O3 est préparé par imprégnation d'une poudre d'alumine de surface spécifique de 180 m²/g. Ce support se présente sous forme de poudre de granulométrie comprise entre 10 et 150 microns.
Après imprégnation, le support est séché et calciné à 400°C.
La teneur finale en cobalt est de 12,5 %.

### Exemple 4 : Tests catalytiques

Les catalyseurs A, B et C décrits dans les exemples 1 et 2 ci-dessus sont testés dans un réacteur opérant en phase liquide, avec le catalyseur en suspension (slurry). Le réacteur est parfaitement agité, fonctionne en continu et opére avec une concentration de 10 % en volume de catalyseur dans la suspension.
Les conditions de test sont les suivantes :
- Température = 230°C,
- Pression = 2MPa
- vitesse volumique horaire (VVH) = 1000 h⁻¹
rapport molaire H₂/CO = 2/1

**Table 1 :**

| **Conversion du gaz de synthèse en hydrocarbures** | | | | |
|---|---|---|---|---|
| **Catalyseur** | **Conv CO (%vol après 100h)** | **Distribution des produits formés (%poids)** | | |
| | | **C1** | **C1-C4** | **C5+** |
| A (invention) | 56 | **9** | **25** | **66** |
| B (invention) | 46 | **8** | **23** | **69** |
| C (invention) | 45 | **12** | **27** | **61** |

Les résultats montrent que les catalyseurs A et B selon l'invention conduisent à une formation d'hydrocarbures C5+ plus importante que le catalyseur comparatif C.

### Exemple 5 : Tests catalytiques en colonne à bulle

Les catalyseurs décrits dans les exemples 1 et 2 sont mis en oeuvre dans une colonne à bulle triphasique de diamètre interne 50 mm et hauteur 1500 mm. Cette colonne est également équipée d'une tubulure d'admission du gaz de synthèse, en bas de colonne, d'une tubulure de soutirage de la suspension au dessus du niveau liquide, d'une tubulure de réinjection de la suspension en bas de colonne, et d'une boucle de circulation comprenant un dégazeur, un décanteur, et une pompe.

Le catalyseur A est introduit dans la colonne à raison de 500 g dans 1,5 I de paraffine n- C18, et utilisé dans les conditions suivantes :
débit de charge : 1 m³/h de mélange CO + H2
rapport molaire H2/CO de 1/2
température : 220°C et pression totale 20 bars.

La conversion du CO de 72 %, et la sélectivité en hydrocarbures C5+ de 78 %. Le catalyseur est séparé des produits liquides au moyen du décanteur, la teneur en catalyseur dans le liquide après décantation est inférieure à 100 ppm poids.

Le catalyseur C est mis en oeuvre dans les mêmes conditions que le catalyseur A. Il conduit à une conversion du CO de 61 %, et une sélectivité en hydrocarbures C5+ de 72 %. Après décantation dans le même appareil, le liquide obtenu contient 0,15 % poids de catalyseur, ce qui oblige à effectuer une filtration à l'issue de la décantation.

Ces exemples montrent l'intérêt du catalyseur utilisé dans le procédé selon l'invention dans une mise en oeuvre en slurry. Le catalyseur utilisé dans le procédé selon l'invention conduit en effet à une formation plus importante d'hydrocarbures C5+. Il est par ailleurs plus aisément séparé des produits liquides au moyen d'une technique simple telle que par exemple la décantation.

## Revendications

1. Procédé de synthèse d'hydrocarbures à partir de gaz de synthèse, en présence d'un catalyseur constitué par un alliage métallique de Raney dispersé dans une phase liquide, dans lequel le catalyseur comprend au moins au moins 60 % poids d'au moins un métal choisi parmi les métaux des groupes 8, 9 ou 10.

2. Procédé selon la revendication 1 dans lequel le métal est choisi parmi le fer ou le cobalt.

3. Procédé selon l'une des revendications 1 ou 2 comprenant en outre au moins un élément additionnel choisi parmi les métaux alcalins et les métaux des groupes 4, 5, 6, 7, 8, 9, 10 ou 11.

4. Procédé selon la revendication 3 dans lequel l'élément additionnel est choisi dans le groupe constitué par le titane, le zirconium, le fer, le ruthénium, le molybdène, le tungstène et le tantale.

5. Procédé selon l'une des revendications 3 ou 4 dans lequel la teneur en poids de l'élément additionnel par rapport au poids total de catalyseur est comprise entre 0 et environ 12 % poids.

6. Procédé selon l'une des revendication 1 à 5 dans lequel le catalyseur présente une taille de grain inférieure à environ 700 microns.

7. Procédé selon l'une des revendications 1 à 6 dans lequel la phase liquide est une coupe kérosène ou gasoil issue de la synthèse Fischer-Tropsch.

8. Procédé selon l'une quelconque des revendications 1 à 7 dans lequel le catalyseur est modifié au moyen d'un pré traitement avant l'introduction dans le réacteur.

9. Procédé selon la revendication 8 dans lequel le pré traitement est une réduction effectuée à une température comprise entre 100°C et 600°C, à une pression comprise entre 0,1 et 10 MPa et à une vitesse volumétrique horaire entre 100 et 40 000 volumes de mélange par volume de catalyseur et par heure.

10. Procédé selon l'une quelconque des revendications 1 à 9 dans lequel le catalyseur est mis en oeuvre dans un réacteur de type colonne à bulle triphasique.

## Claims

1. A process for synthesising hydrocarbons from synthesis gas, in the presence of a catalyst constituted by a Raney metal alloy dispersed in a liquid phase, in which the catalyst comprises at least 60% by weight of at least one metal selected from metals from groups 8, 9 and 10.

2. A process according to claim 1, in which the metal is selected from iron or cobalt.

3. A process according to claim 1 or claim 2, further comprising at least one additional element selected from alkali metals and metals from groups 4, 5, 6, 7, 8, 9, 10 and 11.

4. A process according to claim 3, in which the additional element is selected from the group formed by titanium, zirconium, iron, ruthenium, molybdenum, tungsten and tantalum.

5. A process according to claim 3 or claim 4, in which the amount of additional element with respect to the total weight of catalyst is in the range 0 to about 12% by weight.

6. A process according to any one of claims 1 to 5, in which the grain size of the catalyst is less than about 700 microns.

7. A process according to any one of claims 1 to 6, in which the liquid phase is a kerosene or gas oil cut from the Fischer-Tropsch synthesis.

8. A process according to any one of claims 1 to 7, in which the catalyst is modified by means of a pre-treatment before introducing it into the reactor.

9. A process according to claim 8, in which the pre-treatment is a reduction carried out at a temperature in the range 100°C to 600°C, at a pressure in the range 0.1 to 10 MPa and at an hourly space velocity in the range 100 to 40000 volumes of mixture per volume of catalyst per hour.

10. A process according to any one of claims 1 to 9, in which the catalyst is used in a slurry bubble column type reactor.

## Patentansprüche

1. Verfahren zur Synthese von Kohlenwasserstoffen ausgehend von Synthesegas in Gegenwart eines Katalysators, der aus einer Raney-Metall-Legierung besteht, die in einer Flüssigphase dispergiert ist, in welchem der Katalysator wenigstens 60 Gewichtsprozent wenigstens eines Metalls, gewählt unter den Metallen der Gruppen 8, 9 oder 10, umfasst.

2. Verfahren nach Anspruch 1, bei dem das Metall unter Eisen oder Kobalt gewählt wird.

3. Verfahren nach einem der Ansprüche 1 oder 2, im übrigen wenigstens ein Zusatzelement umfassend, das unter den Alkalimetallen und den Metallen der Gruppen 4, 5, 6, 7, 8, 9, 10 oder 11 gewählt ist.

4. Verfahren nach Anspruch 3, bei dem das Zusatzelement aus der Gruppe gewählt ist, die gebildet wird durch Titan, Zirkonium, Eisen, Ruthenium, Molybdän, Wolfram und Tantal.

5. Verfahren nach einem der Ansprüche 3 oder 4, bei dem der Gewichtsgehalt des Zusatzelements im Verhältnis zum Gesamt-Katalysator-Gewichtsgehalt zwischen 0 und etwa 12 Gewichtsprozent liegt.

6. Verfahren nach einem der Ansprüche 1 - 5, bei dem der Katalysator eine Korngröße unter etwa 700 µm aufweist.

7. Verfahren nach einem der Ansprüche 1 - 6, bei dem die Flüssigphase eine Kerosin- oder Dieselfraktion der Fischer-Tropsch-Synthese ist.

8. Verfahren nach einem der Ansprüche 1 - 7, bei dem der Katalysator mittels einer Vorbehandlung vor Einführung in den Reaktor modifiziert wird.

9. Verfahren nach Anspruch 8, bei dem die Vorbehandlung eine Reduktion ist, die bei einer Temperatur zwischen 100°C und 600°C, bei einem Druck zwischen 0,1 und 10 MPa und einer stündlichen Volumengeschwindigkeit zwischen 100 und 40.000 Mischungsvolumen pro Katalysator-Volumen und pro Stunde durchgeführt wird.

10. Verfahren nach einem der Ansprüche 1 - 9, bei dem der Katalysator in einem Reaktor vom triphasischen Kolonnentyp mit Blasen eingesetzt wird.
